# EUROPÄISCHE PATENTANMELDUNG

(11) **EP 1 698 287 A2**
(43) Veröffentlichungstag der Anmeldung: **06.09.2006**
(21) Anmeldenummer: 06110494.9
(22) Anmeldetag: 28.02.2006
(51) Int. Cl.: A61B 17/22

(54) **Stoßwellensystem**

(30) Priorität: 01.03.2005 DE 102005009904
(71) Anmelder: SIEMENS AKTIENGESELLSCHAFT, 80333 München (DE)
(72) Erfinder: Lanski, Markus, 90408 Nürnberg (DE); Meinert, Christian, 91080 Marloffstein (DE)

(57) **Zusammenfassung**

Die Erfindung betrifft ein Stoßwellensystem mit einer Stoßwellenquelle, die Stoßwellen für eine Behandlung eines Patienten erzeugt, wobei zu Beginn der Behandlung die Stoßwellenenergie, ausgehend von einem vorgebbaren Energie-Anfangswert, automatisch in vorgebbaren Energiestufen sukzessive auf einen vorgebbaren Energie-Endwert erhöht wird. Ein derartiges Stoßwellensystem ermöglicht einen optimierten Beginn einer Stoßwellenbehandlung.

## Beschreibung

Die Erfindung betrifft ein Stoßwellensystem mit einer Stoßwellenquelle, die Stoßwellen für eine Behandlung eines Patienten erzeugt.

Ein derartiges Stoßwellensystem dient zur Behandlung mit extrakorporalen Stoßwellen, meistens in der Lithotripsie und in der Schmerztherapie, insbesondere in der extrakorporalen Stoßwellentherapie (ESWT).

Bei der Lithotripsie handelt es sich um eine therapeutische Methode, ein im Körper eines Lebewesens befindliches Konkrement (z.B. Gallenstein, Nierenstein) ohne operativen Eingriff mit Hilfe von fokussierten Stoßwellen zu zerstören. Sowohl in der Lithotripsie als auch in der extrakorporalen Stoßwellentherapie wird zu Beginn der Behandlung mit niedrigen Energiewerten begonnen, um den Patienten an die Behandlung und die damit möglicherweise verbundenen Schmerzen zu gewöhnen. Dieses langsame Erhöhen der Energiewerte, das der Anwender durchführt, wird als Ramping bezeichnet. Ziel ist es, für die jeweilige Applikation eine möglichst hohe Energiestufe zu erreichen, um eine effektive Therapie zu gewährleisten.

Der Erfindung liegt die Aufgabe zugrunde, ein Stoßwellensystem der eingangs genannten Art zu schaffen, das einen optimierten Beginn einer Stoßwellenbehandlung ermöglicht.

Diese Aufgabe wird erfindungsgemäß durch ein Stoßwellensystem mit den Merkmalen des Anspruchs 1 gelöst. Vorteilhafte Ausgestaltungen der Erfindung sind jeweils Gegenstand von weiteren Ansprüchen.

Das Stoßwellensystem nach Anspruch 1 umfasst eine Stoßwellenquelle, die Stoßwellen für eine Behandlung eines Patienten erzeugt, wobei zu Beginn der Behandlung die Stoßwellenenergie, ausgehend von einem vorgebbaren Energie-Anfangswert, automatisch in vorgebbaren Energiestufen sukzessive auf einen vorgebbaren Energie-Endwertwert erhöht wird.

Das Stoßwellensystem gemäß Anspruch 1 ermöglicht einen optimierten Beginn der Stoßwellenbehandlung beim Patienten, da die Energiestufen wesentlich feiner als bei einer manuellen Erhöhung ausgeprägt sein können. Hierzu wird bei jeder einzelnen Stoßwelle die Hochspannung, welche den Energiewert bestimmt, nur um einen sehr kleinen Betrag erhöht. Damit sind Änderungen möglich, die nur einem Bruchteil einer konventionellen (manuellen) Erhöhung der Energiewerte entsprechen. Dadurch ist eine sehr schonende Gewöhnung des Patienten an den notwendigen Energie-Endwert möglich, da ein sprungweises Erhöhen der Energiewerte sowie ein damit verbundenes starkes Ansteigen des Schmerzempfindens zuverlässig vermieden wird.

Da die Erhöhung auf den Energie-Endwertwert automatisch erfolgt, müssen vom Anwender lediglich die Parameter für die Energiestufen vorgegeben werden (dies kann z.B. bei der Inbetriebnahme des Stoßwellensystems erfolgen) und das Stoßwellensystem bei einem vorgebbaren Energie-Anfangswert gestartet werden. Eingriffe des Anwenders sind bis zum Erreichen des Energie-Endwertes in der Regel nicht mehr erforderlich.

Die Erhöhung der Stoßwellenenergie ist im Rahmen der Erfindung in vielfältiger Weise parametrierbar. So kann z.B. gemäß Anspruch 2 der vorgebbare Energie-Anfangswert, bei dem die Behandlung begonnen wird, konstant, insbesondere Null, sein.

Gemäß einer weiteren Ausführungsform nach Anspruch 3 ist der Energie-Anfangswert in Abhängigkeit von dem bei der letzten Behandlung applizierten Energie-Endwert vorgebbar. Damit kann - falls beim Patienten bereits eine gewisse Gewöhnung an die Stoßwellen erreicht wurde - mit einem höheren Energie-Anfangswert als dem minimalen Energie-Anfangswert begonnen werden. Die Zeit bis zum Erreichen des für eine erfolgreiche Therapie erforderlichen Energie-Endwertes kann dadurch deutlich verkürzt werden.

Bei einem Stoßwellensystem gemäß Anspruch 4 ist die sukzessive Erhöhung der Stoßwellenenergie derart feinstufig durchführbar, dass die Erhöhung im Wesentlichen einen stetigen Verlauf aufweist. Je feinstufiger die Erhöhung der Stoßwellenenergie durchgeführt wird, desto schonender ist die Gewöhnung des Patienten an den für die Applikation notwendigen Energie-Endwert möglich.

Dieser Verlauf der Erhöhung der Stoßwellenenergie kann hierbei, wie in Anspruch 5 angegeben, im Wesentlichen eine Gerade bilden. Im Rahmen der Erfindung sind bei der Erhöhung der Stoßwellenenergie jedoch auch andere, von einer Geraden abweichende Verläufe möglich.

Ist der Verlauf der Erhöhung der Stoßwellenenergie - wie bei einer Ausgestaltung nach Anspruch 6 - durch eine vorgebbare Funktion definiert, dann kann gemäß Anspruch 7 diese Funktion beispielsweise in Abhängigkeit von dem bei der Behandlung applizierten Energie-Endwert vorgegeben werden.

Bei einer vorteilhaften Ausführungsform der Stoßwellensystems nach Anspruch 8 schaltet die automatische Erhöhung der Stoßwellenenergie bei einem manuellen Eingriff ab und wechselt in einen manuellen Modus.

Weiterhin ist es im Rahmen der Erfindung möglich, dass - wie in Anspruch 9 beschrieben - die Stoßwellenenergie nach dem Erreichen des bei der letzten Behandlung applizierten Energie-Endwertes manuell auf einen neuen Energie-Endwert einstellbar ist.

Eine besonders benutzerfreundliche Variante des erfindungsgemäßen Stoßwellensystems stellen die Ausgestaltungen nach den Ansprüchen 10 und 11 dar, bei denen die Werte und/oder die Zwischenwerte der Energiestufen anzeigbar sind, bzw. der Verlauf der Erhöhung der Stoßwellenenergie und/oder Verlauf der Zwischenwerte grafisch darstellbar ist.

Durch die vorgenannten Parametrierungsmöglichkeiten lässt sich der Verlauf der Erhöhung der Stoßwellenenergie auf einfache Weise an unterschiedliche Behandlungsmethoden und an das unterschiedliche Schmerzempfinden einzelner Patienten anpassen. Auch anwenderspezifische Applikationsvarianten sind damit einfach realisierbar. Diese Parametrisierung kann sowohl einmalig durch eine Serviceeinstellung bei der Erstinbetriebnahme bzw. bei Wartungsarbeiten als auch durch den Anwender selbst erfolgen.

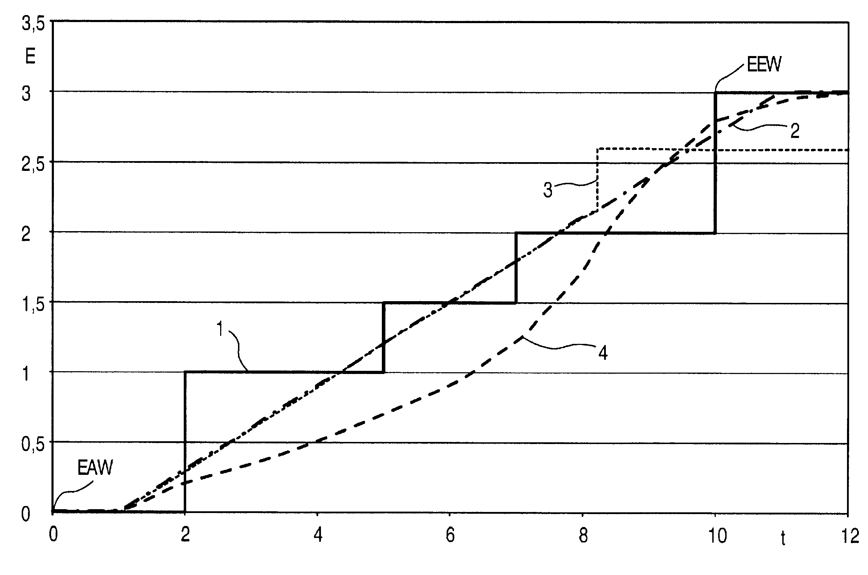

Nachfolgend wird anhand von verschiedenen Einstellungen des erfindungsgemäßen Stoßwellensystems die Erfindung in der Zeichnung anhand einer einzigen Figur näher erläutert.

In der Zeichnung ist auf der Abszissenachse jeweils die Zeit t als dimensionslose Größe aufgetragen. Weiterhin ist auf der Ordinatenachse jeweils die Stoßwellenenergie E als dimensionslose Größe aufgetragen.

Ein möglicher Verlauf einer manuellen Erhöhung der Stoßwellenenergie E ist mit 1 bezeichnet. Drei Beispiele für mit dem erfindungsgemäßen Stoßwellensystem mögliche Verläufe einer automatischen Erhöhung der Stoßwellenenergie E sind mit 2, 3 und 4 bezeichnet.

Die in der Zeichnung dargestellte Behandlung wird bei einem Energie-Anfangswert EAW = 0 bei t = 0 manuell begonnen, wobei die Energiestufen unterschiedliche Höhen sowie unterschiedliche Breiten aufweisen.

Zum Zeitpunkt t = 2 wird die Stoßwellenenergie auf einen Wert E = 1 erhöht. Bei t = 5 wird die Stoßwellenenergie auf E = 1,5 und bei t = 7 auf 2 erhöht. Bei t = 10 wird die Stoßwellenenergie auf ihren Energie-Endwert EEW = 3 angehoben.

Gegenüber der manuellen Erhöhung der Stoßwellenenergie erfolgt die automatische Erhöhung der Stoßwellenenergie bei dem erfindungsgemäßen Stoßwellensystem sukzessive und derart feinstufig, dass die Erhöhung im Wesentlichen einen stetigen Verlauf 2 aufweist. Im dargestellten Ausführungsbeispiel bildet der Verlauf 2 der Erhöhung der Stoßwellenenergie E im Wesentlichen eine Gerade.

Der gewählte Verlauf 2 beginnt bei t = 0 mit einem Energie-Anfangswert, der bis zum Zeitpunkt t = 1 bei EAW = 0 liegt und dann etwa eine Steigung von 0,3 aufweist. Der Energie-Endwert EEW = 3 wird etwa bei t = 11,5 erreicht.

Aus dem Vergleich der beiden Verläufe 1 und 2 ist ersichtlich, dass eine Erhöhung der Stoßwellenenergie gemäß Verlauf 2 schonender für den Patienten ist als eine Erhöhung gemäß Verlauf 1.

Der Verlauf 3 der Stoßwellenenergie E unterscheidet sich vom Verlauf 2 dadurch, dass der Anwender zum Zeitpunkt t = 8,1 und bei einer Stoßwellenenergie von E = 2,1 eine manuelle Erhöhung auf einen Energie-Endwert EEW = 2,6 vornimmt.

Der Verlauf 4 der Stoßwellenenergie E, der von den Verläufen 2 und 3 abweicht, ist insbesondere bei Patienten mit einem starken Schmerzempfinden, beispielsweise Kinder und ältere Personen, vorteilhaft.

Der Verlauf 4 beginnt mit einem Energie-Anfangswert, der bis zum Zeitpunkt t = 1 bei EAW = 0 liegt und dann bis zu einer Stoßwellenenergie von E = 2,4 zum Zeitpunkt t = 9 ständig unterhalb der Stoßwellenenergie gemäß dem Verlauf 2 bleibt. Der Energie-Endwert EEW = 3 wird erst bei t = 12 - und damit später als bei dem Verlauf 2 der Stoßellenenergie E - erreicht.

Aus der Erläuterung der nicht einschränkenden Ausführungsbeispiele der Erfindung wird deutlich, dass mit dem erfindungsgemäßen Stoßwellensystem auf einfache Weise ein optimierter Beginn einer Stoßwellenbehandlung möglich ist.

## Patentansprüche

1. Stoßwellensystem mit einer Stoßwellenquelle, die Stoßwellen für eine Behandlung eines Patienten erzeugt, wobei zu Beginn der Behandlung die Stoßwellenenergie, ausgehend von einem vorgebbaren Energie-Anfangswert, automatisch in vorgebbaren Energiestufen sukzessive auf einen vorgebbaren Energie-Endwert erhöht wird.

2. Stoßwellensystem nach Anspruch 1, wobei der Energie-Anfangswert konstant, insbesondere Null, ist.

3. Stoßwellensystem nach Anspruch 1, wobei der Energie-Anfangswert in Abhängigkeit von dem bei der letzten Behandlung applizierten Energie-Endwert vorgebbar ist.

4. Stoßwellensystem nach Anspruch 1, wobei die sukzessive Erhöhung der Stoßwellenenergie derart feinstufig durchführbar ist, dass die Erhöhung im Wesentlichen einen stetigen Verlauf aufweist.

5. Stoßwellensystem nach Anspruch 4, wobei der Verlauf der Erhöhung der Stoßwellenenergie im Wesentlichen eine Gerade bildet.

6. Stoßwellensystem nach Anspruch 3 oder 4, wobei der Verlauf der Erhöhung der Stoßwellenenergie durch eine vorgebbare Funktion definiert ist.

7. Stoßwellensystem nach Anspruch 6, wobei die Funktion in Abhängigkeit von dem bei der letzten Behandlung applizierten Energie-Endwert vorgebbar ist.

8. Stoßwellensystem nach Anspruch 1, wobei die automatische Erhöhung der Stoßwellenenergie bei einem manuellen Eingriff abschaltet und in einen manuellen Modus wechselt.

9. Stoßwellensystem nach Anspruch 1, wobei die Stoßwellenenergie nach dem Erreichen des bei der letzten Behandlung applizierten Energie-Endwertes manuell auf einen neuen Energie-Endwert einstellbar ist.

10. Stoßwellensystem nach Anspruch 1, wobei die Werte und/oder die Zwischenwerte der Energiestufen anzeigbar sind.

11. Stoßwellensystem nach Anspruch 1, wobei der Verlauf der Erhöhung der Stoßwellenenergie und/oder der Verlauf der Zwischenwerte grafisch darstellbar ist.
